# FASCICULE DE BREVET EUROPEEN

(11) **EP 3 452 151 B1**
(45) Date de publication et mention de la délivrance du brevet: **25.03.2020**
(21) Numéro de dépôt: 17727281.2
(22) Date de dépôt: 02.05.2017
(51) Int. Cl.: A61M 15/00, A61M 11/00, B05B 11/02, B05B 11/00, A61M 5/31

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 04.05.2016 FR 1654057
(43) Date de publication de la demande: 13.03.2019
(73) Titulaire: Aptar France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2017/051036
(87) Numéro de publication internationale: WO 2017/191400

(56) Documents cités:
- EP-A1- 0 546 607
- EP-A1- 1 504 783
- EP-A1- 2 896 421
- WO-A1-99/28042
- WO-A1-02/102443
- WO-A1-2010/139883
- DE-A1- 19 700 437
- DE-U1- 20 022 559
- FR-A1- 2 970 955
- GB-A- 2 412 326
- US-B1- 6 321 942
- US-B1- 6 708 846
- US-B2- 8 734 392

## Description

La présente invention concerne un dispositif de distribution de produit fluide, en particulier un dispositif du type unidose ou bidose, c'est-à-dire contenant seulement une ou deux doses de produit fluide.

Les dispositifs unidoses et bidoses sont bien connus. Ils comportent généralement un réservoir contenant une ou deux doses de produits fluide et une tête de distribution mobile par rapport audit réservoir pour distribuer le produit fluide, notamment via un piston coulissant dans ledit réservoir. Le réservoir peut être assemblé dans un corps, avec ladite tête assemblée sur ledit corps. Les documents US8734392 et FR2970955 décrivent des dispositifs de ce type. Pour éviter les actionnements accidentels ou non souhaités, des moyens de blocage sont généralement prévus, comme par exemple des ponts sécables, qu'il faut surmonter et/ou casser pour réaliser l'actionnement. De même, des moyens de précompression sont aussi généralement prévus, pour assurer que l'effort qui est nécessaire pour surmonter ces moyens de précompression fournit assez d'énergie pour garantir la course d'actionnement complète et donc pour assurer la distribution de la dose complète. De manière avantageuse, ces moyens de blocage et ces moyens de précompression peuvent être formés par les mêmes moyens, typiquement des ponts sécables.

Un inconvénient avec ces dispositifs de l'art antérieur concerne l'actionnement qui peut s'avérer difficile, notamment lorsque des moyens de précompression et/ou de blocage, tels que par exemple des ponts sécables, sont prévus. Ceci est d'autant plus vrai que les dimensions radiales du réservoir et/ou du corps sont petites, en particulier au niveau de la surface d'appui manuel de l'utilisateur. Un autre inconvénient concerne le risque dysfonctionnement ou de dose incomplète en cas d'actionnement qui ne serait pas axial.

Les documents US6708846, WO02102443, EP0546607, EP1504783, DE20022559, EP2896421, WO9928042, DE19700437, US6321942, GB2412326 et WO2010139883 décrivent des dispositifs de l'état de la technique.

La présente invention a pour but de fournir un dispositif qui ne reproduit pas les inconvénients susmentionnés.

La présente invention a aussi pour but de fournir un dispositif qui facilite l'actionnement.

La présente invention a également pour but de fournir un dispositif qui est simple et peu coûteux à fabriquer et à assembler.

La présente a donc pour objet un dispositif de distribution de produit fluide comportant un corps supportant fixement un réservoir contenant du produit fluide, une tête de distribution étant assemblée sur ledit corps en étant axialement déplaçable par rapport audit réservoir, ladite tête de distribution étant pourvue d'un orifice de distribution, ladite tête de distribution comportant une surface d'appui proximale recevant lors de l'actionnement au moins un doigt, typiquement deux doigts, de l'utilisateur, et ledit corps comportant une surface d'appui distale pouvant recevoir lors de l'actionnement un doigt, typiquement le pouce, de l'utilisateur, ledit dispositif comportant un organe d'actionnement amovible qui se fixe de manière amovible sur ledit corps au niveau de ladite surface d'appui distale, ledit organe d'actionnement amovible ayant une dimension radiale supérieure à celle de ladite surface d'appui distale, ledit organe d'actionnement amovible comportant un manchon axial creux qui s'emmanche de manière amovible, notamment par frottements, sur ledit corps, notamment autour de ladite surface d'appui distale, ledit organe d'actionnement amovible comportant une bride radiale, tel qu'un disque, formant une surface d'appui de substitution, qui se projette radialement vers l'extérieur par rapport à ladite surface d'appui distale dudit corps.

Avantageusement, ledit réservoir contient une seule dose de produit fluide.

En variante, ledit réservoir contient seulement deux doses de produit fluide.

Avantageusement, ledit organe d'actionnement amovible est réalisé dans un matériau souple, tels qu'un thermoplastique élastomère (TPE).

Avantageusement, des moyens de blocage et/ou de précompression sont prévus entre ledit corps et ladite tête de distribution.

Avantageusement, lesdits moyens de blocage et/ou de précompression comportent des ponts sécables.

Avantageusement, l'aire de ladite surface d'appui distale est inférieure à 100 mm², avantageusement inférieure à 80 mm², de préférence environ 60 mm².

Avantageusement, l'aire dudit organe d'actionnement amovible est supérieure à 150 mm², avantageusement supérieure à 220 mm², de préférence environ 515 mm².

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective d'un dispositif de distribution auquel s'applique l'invention,
- la figure 2 est une vue de détail en perspective de l'organe d'actionnement amovible selon un mode de réalisation avantageux,
- la figure 3 est une vue schématique en section transversale du dispositif de la figure 1 pourvu de l'organe d'actionnement amovible de la figure 2, avant actionnement, et
- la figure 4 est une vue similaire à celle de la figure 3, après actionnement.

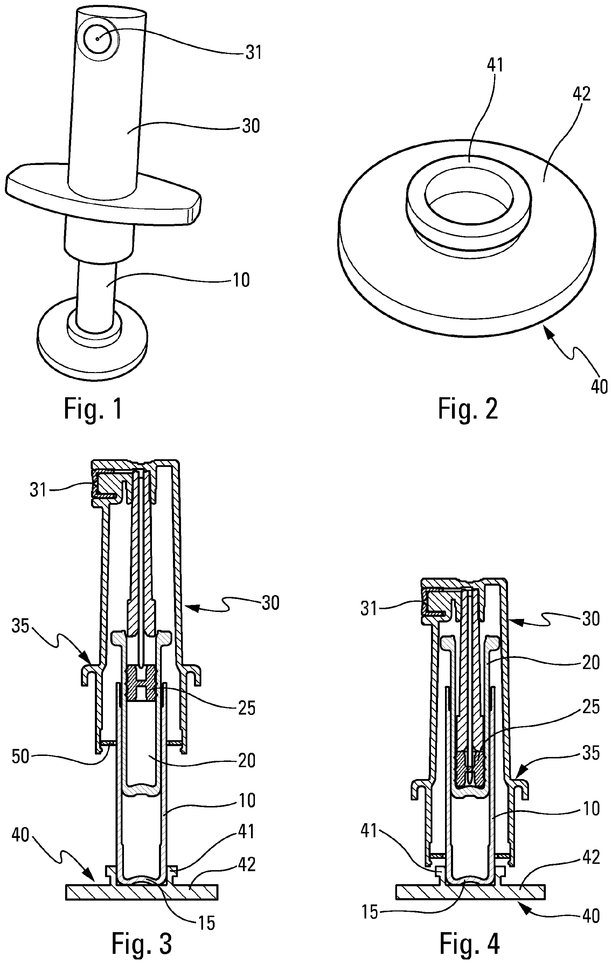

Dans la description, les termes "axial" et "radial" se réfèrent à l'axe longitudinal du dispositif représenté sur les figures 3 et 4. Les termes "proximal" et "distal" se réfèrent à l'orifice de distribution.

Le dispositif de distribution de produit fluide selon l'invention comporte classiquement un corps 10 supportant fixement un réservoir 20 contenant du produit fluide. Une tête de distribution 30 est assemblée sur ledit corps 10 de manière axialement déplaçable par rapport audit réservoir 20, ladite tête de distribution 30 étant pourvue d'un orifice de distribution 31.

De manière connue, ladite tête de distribution 30 comporte une surface d'appui proximale 35 recevant lors de l'actionnement au moins un doigt, typiquement deux doigts, de l'utilisateur. De même, le corps 10 comporte une surface d'appui distale 15 pouvant recevoir lors de l'actionnement un doigt, typiquement le pouce, de l'utilisateur.

Un piston 25 est monté coulissant dans le réservoir 20, ledit piston 25 étant, lors de l'actionnement, déplacé par la tête de distribution 30 lorsque celle-ci se déplace axialement par rapport au corps 10 et donc par rapport au réservoir 20.

Selon l'invention, le dispositif comporte un organe d'actionnement amovible 40 qui coopère de manière amovible avec ledit corps 10 au niveau de ladite surface d'appui distale 15. En particulier, l'organe d'actionnement amovible 40 se fixe de manière amovible sur ledit corps 10. Cet organe d'actionnement amovible 40 a une dimension radiale supérieure à celle de ladite surface d'appui distale 15. Avantageusement, l'aire de ladite surface d'appui distale 15 est inférieure à 100 mm², en particulier inférieure à 80 mm², notamment environ 60 mm², alors que l'aire dudit organe d'actionnement amovible est avantageusement supérieure à 150 mm², en particulier supérieure à 220 mm², notamment environ 515 mm². Ceci augmente donc la surface de contact entre le pouce de l'utilisateur et le dispositif lors de l'actionnement, facilitant ainsi l'actionnement du dispositif en répartissant la force d'actionnement exercée par le pouce sur une plus grande surface d'appui. En rendant l'effort d'actionnement plus acceptable, le dispositif de l'invention permet aussi de mieux maîtriser la course d'actionnement complète. Ceci peut être particulièrement intéressant dans le cas d'une dose nominale élevée, due par exemple à une course d'actionnement importante ou à un produit visqueux.

L'organe d'actionnement amovible 40 comporte un manchon axial creux 41 qui s'emmanche de manière amovible, notamment par frottements, sur ledit corps 10. Comme visible sur les figures 1, 3 et 4, cet emmanchement se fait de préférence autour de la surface d'appui distale 15 du corps 10. Eventuellement, ledit manchon axial creux 41 peut comporter des profils de serrage, tels que des nervures ou similaire, pour favoriser une mise en place et un retrait aisé dudit organe d'actionnement amovible 40.

L'organe d'actionnement amovible 40 comporte une bride radiale 42, tel qu'un disque, formant la surface d'appui de substitution, qui se projette radialement vers l'extérieur par rapport à la surface d'appui distale 15 du corps 10, augmentant ainsi la zone de contact avec le pouce.

Pour éviter un actionnement non souhaité et pour générer une précompression dans la main de l'utilisateur lors de l'actionnement, le dispositif comporte avantageusement des moyens de blocage et/ou de précompression 50, de préférence formés par des ponts sécables disposés entre le corps 10 et la tête 30. Bien entendu, d'autres moyens de blocage et/ou de précompression sont envisageables. De plus, les moyens de précompression pourraient être séparés des moyens de blocage.

L'ajout d'un organe d'actionnement amovible 40 sur la base du corps 10 rend l'effort à fournir par l'utilisateur plus acceptable du fait de la surface plus importante en contact avec le pouce de l'utilisateur.

Ledit organe d'actionnement 40 étant amovible du corps, l'utilisateur a le choix de l'utiliser ou de ne pas l'utiliser.

Ledit organe d'actionnement amovible 40 est avantageusement réalisé en un matériau relativement souple, tel qu'un TPE (thermoplastique élastomère). Ceci présente plusieurs avantages, dont notamment :
- un contact agréable avec le pouce de l'utilisateur,
- un assemblage simple sur le corps 10 et une tenue par serrage également simple.

Ledit organe d'actionnement amovible 40 présente également les avantages suivants par rapport à une solution qui serait non amovible du corps 10 :
- l'organe d'actionnement amovible 40 se démonte facilement après actionnement, de sorte que si l'on tire dessus, il se détache du corps 10; on ne peut donc pas s'en servir pour démonter le dispositif et ainsi avoir accès au produit fluide restant dans le dispositif après actionnement,
- la souplesse de la matière implique que ledit organe d'actionnement amovible 40 se détache du corps 10 si l'utilisateur ne pousse pas dans l'axe du corps; ceci permet d'éviter le tiltage et donc limite les risques de dysfonctionnement ou de dose incomplète.

L'invention s'applique plus particulièrement à des dispositifs du type unidose ou bidose, contenant seulement une seule ou seulement deux doses de produit fluide.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant un corps (10) supportant fixement un réservoir (20) contenant du produit fluide, une tête de distribution (30) étant assemblée sur ledit corps (10) en étant axialement déplaçable par rapport audit réservoir (20), ladite tête de distribution (30) étant pourvue d'un orifice de distribution (31), ladite tête de distribution (30) comportant une surface d'appui proximale (35) recevant lors de l'actionnement au moins un doigt, typiquement deux doigts, de l'utilisateur, et ledit corps (10) comportant une surface d'appui distale (15) pouvant recevoir lors de l'actionnement un doigt, typiquement le pouce, de l'utilisateur, **caractérisé en ce que** ledit dispositif comporte un organe d'actionnement amovible (40) qui se fixe de manière amovible sur ledit corps (10) au niveau de ladite surface d'appui distale (15), ledit organe d'actionnement amovible (40) ayant une dimension radiale supérieure à celle de ladite surface d'appui distale (15), ledit organe d'actionnement amovible (40) comportant un manchon axial creux (41) qui s'emmanche de manière amovible, notamment par frottements, sur ledit corps (10), notamment autour de ladite surface d'appui distale (15), ledit organe d'actionnement amovible (40) comportant une bride radiale (42), tel qu'un disque, formant une surface d'appui de substitution, qui se projette radialement vers l'extérieur par rapport à ladite surface d'appui distale (15) dudit corps (10).

2. Dispositif selon la revendication 1, dans lequel ledit réservoir (20) contient une seule dose de produit fluide.

3. Dispositif selon la revendication 1, dans lequel ledit réservoir (20) contient seulement deux doses de produit fluide.

4. Dispositif selon l'une quelconque des revendications précédentes, dans lequel ledit organe d'actionnement amovible (40) est réalisé dans un matériau souple, tels qu'un thermoplastique élastomère (TPE).

5. Dispositif selon l'une quelconque des revendications précédentes, dans lequel des moyens de blocage et/ou de précompression (50) sont prévus entre ledit corps (10) et ladite tête de distribution (30).

6. Dispositif selon la revendication 5, dans lequel lesdits moyens de blocage et/ou de précompression (50) comportent des ponts sécables.

7. Dispositif selon l'une quelconque des revendications précédentes, dans lequel l'aire de ladite surface d'appui distale (15) est inférieure à 100 mm², avantageusement inférieure à 80 mm², de préférence environ 60 mm².

8. Dispositif selon l'une quelconque des revendications précédentes, dans lequel l'aire dudit organe d'actionnement amovible (40) est supérieure à 150 mm², avantageusement supérieure à 220 mm², de préférence environ 515 mm².

## Patentansprüche

1. Abgabevorrichtung für ein fluidförmiges Produkt, umfassend einen Körper (10), der einen Behälter (20), der ein fluidförmiges Produkt enthält, fest trägt, einen Abgabekopf (30), der auf dem Körper (10) befestigt ist, indem er axial verschiebbar zu dem Behälter (20) ist, wobei der Abgabekopf (30) mit einer Abgabeöffnung (31) versehen ist, wobei der Abgabekopf (30) eine proximale Auflagefläche (35) aufweist, die beim Betätigen mindestens einen Finger, typischerweise zwei Finger, des Benutzers aufnimmt, und wobei der Körper (10) eine distale Auflagefläche (15) aufweist, die beim Betätigen mindestens einen Finger, typischerweise den Daumen, des Benutzers aufnehmen kann, **dadurch gekennzeichnet, dass** die Vorrichtung ein abnehmbares Betätigungsorgan (40) aufweist, das abnehmbar auf dem Körper (10) an der distalen Auflagefläche (15) befestigt wird, wobei das abnehmbare Betätigungsorgan (40) eine radiale Abmessung aufweist, die größer als jene der distalen Auflagefläche (15) ist, wobei das abnehmbare Betätigungsorgan (40) eine hohle axiale Hülse (41) aufweist, die abnehmbar, insbesondere durch Reibung, auf den Körper (10), insbesondere um die distale Auflagefläche (15) herum, aufgesteckt wird, wobei das abnehmbare Betätigungsorgan (40) einen radialen Flansch (42), beispielsweise eine Scheibe, aufweist, der eine Ersatzauflagefläche bildet, die gegenüber der distalen Auflagefläche (15) des Körpers (10) radial nach außen vorsteht.

2. Vorrichtung nach Anspruch 1, wobei der Behälter (20) eine einzige Dosis des fluidförmigen Produktes enthält.

3. Vorrichtung nach Anspruch 1, wobei der Behälter (20) nur zwei Dosen des fluidförmigen Produktes enthält.

4. Vorrichtung nach einem der vorhergehenden Ansprüche, wobei das abnehmbare Betätigungsorgan (40) aus einem flexiblen Material, beispielsweise einem thermoplastischen Elastomer (TPE), hergestellt ist.

5. Vorrichtung nach einem der vorhergehenden Ansprüche, wobei Blockier- und/oder Vorkompressionsmittel (50) zwischen dem Körper (10) und oder dem Abgabekopf (30) vorgesehenen sind.

6. Vorrichtung nach Anspruch 5, wobei die Blockier- und/oder Vorkompressionsmittel (50) abbrechbare Brücken aufweisen.

7. Vorrichtung nach einem der vorhergehenden Ansprüche, wobei die Fläche der distalen Auflagefläche (15) kleiner ist als 100 mm², vorteilhafterweise kleiner als 80 mm², vorzugsweise etwa 60 mm² beträgt.

8. Vorrichtung nach einem der vorhergehenden Ansprüche, wobei die Fläche des abnehmbaren Betätigungsorgans (40) größer ist als 150 mm², vorteilhafterweise größer als 220 mm², vorzugsweise etwa 515 mm² beträgt.

## Claims

1. A fluid dispenser device comprising a body (10) that supports, in stationary manner, a reservoir (20) containing fluid, a dispenser head (30) being assembled on said body (10) so as to be axially movable relative to said reservoir (20), said dispenser head (30) being provided with a dispenser orifice (31), said dispenser head (30) including a proximal bearing surface (35) that during actuation receives at least one, and typically two, of the user's fingers, and said body (10) including a distal bearing surface (15) that during actuation is suitable for receiving one of the user's fingers, and typically the thumb, said device being **characterized in that** it includes a removable actuator member (40) that is fastened in removable manner on said body (10) at said distal bearing surface (15), said removable actuator member (40) having a radial dimension that is greater than the radial dimension of said bearing surface (15), said removable actuator member (40) including a hollow axial sleeve (41) that is engaged in removable manner, in particular by friction, on said body (10), in particular around said distal bearing surface (15), said removable actuator member (40) including a radial flange (42), such as a disk, forming a substitute bearing surface, that projects radially outwards relative to said distal bearing surface (15) of said body (10).

2. A device according to claim 1, wherein said reservoir (20) contains a single dose of fluid.

3. A device according to claim 1, wherein said reservoir (20) contains only two doses of fluid.

4. A device according to any preceding claim, wherein said removable actuator member (40) is made out of a flexible material, such as a TPE.

5. A device according to any preceding claim, wherein blocking and/or precompression means (50) are provided between said body (10) and said dispenser head (30).

6. A device according to claim 5, wherein said blocking and/or precompression means (50) comprise breakable bridges.

7. A device according to any preceding claim, wherein the area of said distal bearing surface (15) is less than 100 mm², advantageously less than 80 mm², preferably about 60 mm².

8. A device according to any preceding claim, wherein the area of said removable actuator member (40) is greater than 150 mm², advantageously greater than 220 mm², preferably about 515 mm².
